# EUROPEAN PATENT APPLICATION

(11) **EP 2 008 624 A1**
(43) Date of publication of application: **31.12.2008**
(21) Application number: 07111056.3
(22) Date of filing: 26.06.2007
(51) Int. Cl.: A61F 5/453

(54) **An adhesive laminate, a male external urinary catheter comprising the adhesive laminate, and a method of applying the adhesive laminate**

(71) Applicant: MBH International A/S, 3450 Allerød (DK)
(72) Inventor: Christensen, Jens Berg, 2450 Copenhagen SV (DK)
(74) Representative: Holme Patent A/S

(57) **Abstract**

An adhesive laminate (1) is adapted for bonding to an inside (15) of an annular section (17) of a male external catheter (7) of a cured elastomeric polymer, preferably a silicone rubber. The adhesive laminate (1) comprises a first adhesive layer (2) directly bonded to the inside (15) of the elastomeric polymer wall (11) and a second adhesive layer (3) for securing the catheter (7) to the skin, and at least one third carrier and/or barrier layer (4) inserted between the first adhesive layer (2) and the second adhesive layer (3). The adhesive laminate can be used as a separate part for adhering a conventional silicone rubber urisheath to the penis or an urisheath may be provided with the adhesive liner as a firmly secured part on the inside of the urisheath. The urisheath can be rolled so that the adhesive layer on the inside face are brought into contact with the outside face of the urisheath without the windings of the rolled urisheath are bonded unreleasbly to each other.

## Description

The present invention relates to an adhesive laminate adapted for bonding to an inside annular section of a male external catheter of a cured elastomeric polymer, the adhesive laminate is of the kind which comprises a first adhesive layer directly adhered to the inside face of the elastomeric polymer wall and a second adhesive layer for securing the catheter to the skin.

External urinary catheters are used by males suffering from urinary incontinence either on a daily basis or in connection with hospital treatment and surgery of urethral disorders. A male external catheter normally comprises a sheath or body portion, such as a tubular body, enclosing the shaft of the penis, and a tip portion that is provided with a comparatively short discharge tube, which, via a hose, is connected to a urine collection bag fastened to e.g. the bed or the leg of the user.

Male external urinary catheters are fixed to the penis by use of a skin adhesive that both bonds to the skin and to the catheter. The adhesive can either be applied to the skin before applying the male external catheter on the penis, or the adhesive can be firmly secured to the male external catheter, e.g. as an adhesive band on the inside of the male external catheter. A male external catheter is traditionally delivered in a rolled-up condition with the adhesive entirely accommodated in the windings.

Some male external catheters are made of latex which induces allergy. To reduce this risk of latex allergy male external catheters are now preferably made of certain thermoplastic elastomers, which however suffers from the disadvantage that they have a tendency to creep over time, thereby giving the tubular body a wavy shape which makes the rolled catheter difficult to unroll.

Silicone is the preferred thermoplastic polymer for manufacturing male external catheters for adhesion to the penis. Silicone is skin friendly and has superb elastic properties which makes unrolling very easy. However, the only adhesives that adhere to fully cured, non-treated silicone are silicone based adhesives. The disadvantage of using such a silicone based adhesive is that when the male external catheter is rolled-up the inside face of the male external catheter carrying the silicone based adhesive comes into contact with the outside face of the male external catheter and bonds with the same strength to the outside as to the inside.

Various known methods addresses this problem and provides methods for producing male external catheters with reduced tendency of that the adhesive on the catheter inside bonds to the catheter outside when the catheter is rolled.

US 5,176,666 describes a common method of applying an acrylic skin adhesive on a male external catheter. The acrylic skin adhesive is of the kind which cannot bond to a cured silicone rubber. To overcome this problem the adhesive is applied to a mandrel and subsequently the mandrel is dipped in a liquid silicone. The adhesive is cross-linked with the silicone using vulcanization. This process reduces the tendency that the adhesive sticks to the outside face of the rolled-up catheter.

US 5,779,964 describes an alternative silicone male external catheter. The male external catheter is made by dipping a mandrel in liquid silicone, curing the silicone, providing the adhesive on the outside face of the cured catheter, curing the adhesive and providing a surface preparation layer on top of the cured adhesive. When the male external catheter is rolled up the surface preparation layer is brought into intimate contact with the inside face of the male external catheter and the adhesive is transferred to the inside face of the male external catheter.

International patent application WO 2006/027349 describes a method for adhering an adhesive to a cured injection molded silicone male external catheter by oxidizing the inside face of the male external catheter using a corona treatment to prepare the inside face to facilitate attachment of a polar pressure sensitive adhesive. A corona treatment is a method for raising the surface tension of a material, e.g. an adhesive, by exposing the air near the material surface to a high-voltage electrical discharge - a corona - that causes the oxygen molecules in the discharge area to divide into their atomic form. These oxygen atoms are then available to bond with the molecules on the surface of the material being treated, thereby changing the molecular surface structure to one that is extremely receptive to adhesives. However, the patent application recognizes that a corona treatment is considered to have a short effect, and additional steps are required to avoid this, thereby making the method expensive and difficult to control. This patent application also describes the use of a special tie-layer of silicone for bonding the adhesive.

All the above-mentioned known methods suffer from the disadvantage that molecules from the skin adhesive migrates into the silicone rubber and vice versa thereby providing the skin adhesive with silicone adhesive properties enabling the skin adhesive to bond to the outside face of the catheter after only a short period of storage. Furthermore, a complete cross-linking of all molecules of an injection-mouldable silicones is difficult to achieve, and molecules which escape cross-linking are still able to adhere to skin adhesive when the catheter is rolled.

For use in the present application the followings terms are to be understood as follows:
- an "adhesive" is a compound that adheres/bonds two items together. Adhesives includes synthetic adhesives based on elastomers, thermoplastic, and thermosetting adhesives. Among these are included pressure sensitive adhesive which are adhesives that forms a bond by the application of pressure to marry the adhesive with the adherend. Once the adhesive and the adherend are in close proximity, molecular interactions contribute to strengthen the bond. Some pressure sensitive adhesive are removable after months or years without leaving residue on the adherend, and they are able to absorb liquid.
- a "silicone adhesive" is a silicone polymer based adhesive, which is able to bond to another silicone polymer material. Silicone adhesives are sticky, flexible and have a high degree of elasticity and are able to serve as a sealant,
- a "laminate" is a multi-layered structure constructed of layers of adhesives or other materials united by adhesives,
- "selectively adheres to a skin surface" means highly specific in receptiveness to a skin surface.

In a first aspect according to the present invention is provided an adhesive laminate of the kind mentioned in the opening paragraph having both an adhesive face which is skin-friendly and an adhesive face which is bondable to an elastomeric polymer.

In a second aspect is provided a male external catheter with non-stick properties to the elastomeric polymer outside face of the catheter.

In a third aspect according to the present invention is provided a male external catheter that remedies the disadvantages and problems of the above mentioned prior art catheters.

In a fourth aspect according to the present invention is provided a male external catheter which do not creep over time, in particular do not creep in the rolled-up state where stretching of the windings takes place.

In a fifth aspect according to the present invention is provided a male external catheter, which do not stick to the clothes of the patient during use.

In a sixth aspect according to the present invention is provided a male external catheter, which manually can be provided with an adhesive laminate.

The novel and unique feature whereby this is achieved according to the present invention is the fact that the adhesive laminate further comprises at least one third carrier and/or barrier layer inserted between the first adhesive layer and the second adhesive layer.

The at least one third carrier and/or barrier layer serves as a backing for both the first and the second adhesive layers to provide structural and dimensional stability which makes the adhesive laminate easy to apply on a penis.

The laminate can be used as a separate part or be provided as a part of a male external catheter.

If the adhesive laminate is selectively mounted, e.g. as an annular band on the penis, the problem that the skin adhesive adheres to the outside face of the catheter is eliminated. The first adhesive layer will provide a firm bond to the inside of the catheter when the catheter is subsequently mounted on top of the laminate and attachment to the skin can be made at exactly the desired location on the penis.

Preferably the elastomeric polymer is a silicone rubber, such as a medical grade silicone rubber, which is known not to induce allergic reactions.

In the preferred embodiment according to the present invention the second adhesive is selected from a group of adhesive materials which selectively adheres to a skin surface but not stick or bond to the elastomeric polymer or sticks to the elastomeric polymer in a releasable manner.

A male external catheter of the kind mentioned in the opening paragraph is prior to use provided in a rolled-up condition and must be able to be unrolled again for use.

The solution to this is according to the present invention to provide a first adhesive layer of the kind which bonds to a cured elastomeric polymer, cover this first adhesive layer with the at least one third carrier and/or barrier layer, and finally provide the second adhesive layer, which is a skin friendly adhesive layer, on top of the at least one third carrier and/or barrier layer. The adhesive laminate may constitute an implemented part of a catheter, in which case the material of the second adhesive must be of a kind which is unable to stick to the elastomeric polymer or sticks to the elastomeric polymer in a releasable manner without leaving residues, so that the catheter without difficulties can be rolled and unrolled again to smoothly cover and stick to the penis for an adequate period. Because the outside face is without adhesive residues the catheter and it will not stick to the patients clothes. In particular the patients underpants can be taken on and off without the catheter sticks to them and pulls the catheter off the penis or causes the catheter to loose its bond to the skin of the penis.

During storage of the male external catheter the at least third carrier and/or barrier layer advantageously prevents molecules from the first adhesive layer from migrating into the second adhesive layer to alter the composition of this second adhesive layer to adopt the adhesive properties of the first adhesive layer so that it is able to bond to the outside of the male external catheter.

In an embodiment which is both inexpensive and simple to manufacture the material of the at least one third carrier and/or barrier layer is an elastic polymer, preferably a thermoplastic polymer, such as for example a polyurethane. However, within the scope of the present invention thermosetting polymers and other cross-linked polymers are foreseen for use in the at least one third carrier and/or barrier layer in the adhesive laminate.

The second adhesive layer may advantageously comprise at least one of the adhesive substances selected from the group comprising acrylic, polyurethane, polyvinyl ether, polyisobutylene, poly(vinylethyl ether), styrenic block copolymer, poly(vinyl-pyrrolidone) adhesives or adhesive formulations including polymer, resin and/or hydrocolloids. Inclusion of a hydrocolloid provides the adhesive laminate with the capability of absorbing liquid without losing the adhesive grip on the skin. These adhesive are preferred due to the skin friendly properties.

Furthermore, any of the first adhesive layer and/or the second adhesive layer may optionally be covered by a release liner, which must be removed before use. The liner prevents unintentional sticking of the adhesive laminate to other surfaces than the surfaces intended for final use.

The present invention also relates to a method of adhering a male external urinary catheter to a penis, where the male external urinary catheter is of the kind comprising a first tubular body part which extends into a second tubular discharge part and adhesion to the penis is achieved by means of the adhesive laminate disclosed above.

The adhesive laminate is advantageously adhered to the penis by means of the second adhesive and subsequently the first adhesive layer of the adhesive laminate is adhered to an inner annular wall section of the male external urinary catheter.

In one embodiment the male external urinary catheter may be detachably arranged on the adhesive laminate to enable change of the catheter without the change of the adhesive laminate.

The present invention also relates to a male external urinary catheter of the kind comprising a first tubular body part provided with an adhesive means for adhering to a penis, said first tubular body part extends into a second tubular discharge part, wherein the adhesive means for adhering the first tubular part to a penis is the adhesive laminate according to the present invention.

The invention will be explained in greater detail below, describing only exemplary embodiments with reference to the drawing, in which
fig. 1 shows seen in perspective a length of an adhesive laminate according to the invention,
fig. 2 is a perspective view of an embodiment of a male external urinary catheter according to the invention,
fig. 3 shows a longitudinal sectional view taken along line III-III of fig. 2, and
fig. 3a shows a part of the sectional view of fig. 3, illustrating in an enlarged scale the composition of the adhesive laminate bonded to the catheter wall.

The adhesive laminate, which is designated in general with the reference numeral 1, consists in the case shown of three layers, a first adhesive layer 2, for adhering to an elastomeric polymer, a second adhesive layer 3 for adhering to a skin surface, and a third carrier and/or barrier layer 4 between the first adhesive layer. The first adhesive layer 2 and the second adhesive layer 3 are protected by release liners 5,6 respectively, to prevent undesired sticking of exposed adhesive surfaces 2,3 to the surroundings.

The dimensions, thicknesses and number of the various layers are only given for illustrative purposes and are not intended to limit the scope of the present invention. The layers may have any suitable thickness depending on the particular purpose and external conditions as well as the height of the laminate may be adjusted.

The adhesive laminate 1 is shown as a cut piece of a band, however within the scope of the present invention the adhesive laminate may be provided in rolls from which a piece of a suitable length is taken. The adhesive laminate may also be provided as an elastic, closed, circumferential band. The release liners are considered optional or can be provided on only one of the layers.

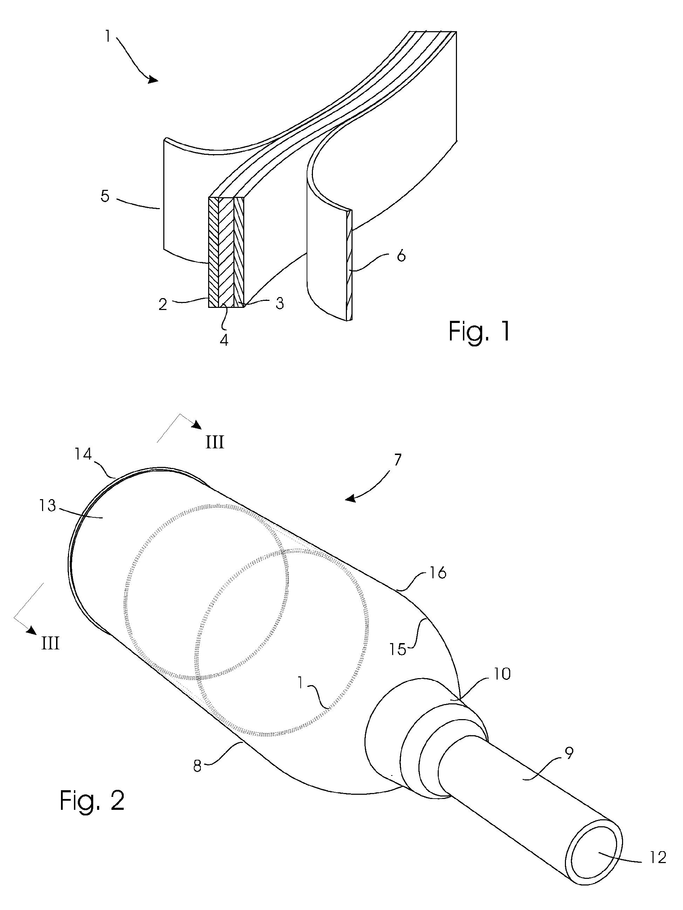

In fig. 2 the adhesive laminate 1 is provided as a part of a male external urinary catheter 7, in the following referred to as an urisheath.

The urisheath 7, which is shown in its unrolled condition, consist of a first tubular body part 8 which extends into a narrower second tubular discharge part 9 for connecting to a urine collecting system, so as to provide a safe and reliable closed urinary bladder drainage system. In the case shown the first tubular part 8 extends into the second tubular discharge part via a transition part 10 having a larger diameter than the second tubular discharge part but a smaller diameter than the first tubular body part to provide the circumferential catheter wall 11 with a stepwise tapering towards the discharge opening 12 of the discharge part 9. The transition part 10 provides the catheter with kink-resistance and can be given other designs. The free opening 13 of the tubular body part 8 opposite the discharge opening 12 has an annular reinforcement rib 14, for enabling rolling of the catheter after manufacturing. The reinforcement rib 14 also facilitate mounting of the urisheath on the penis by rolling the adhesive laminate 1 on the inside 15 of the urisheath 7 into bonding contact with the skin of the penis. After the catheter has been unrolled and mounted on the penis the outside 16 of the urisheath 7 appears free of adhesive residues and the urisheath 7 cannot stick to the clothes of the patient or any other parts of the use environment.

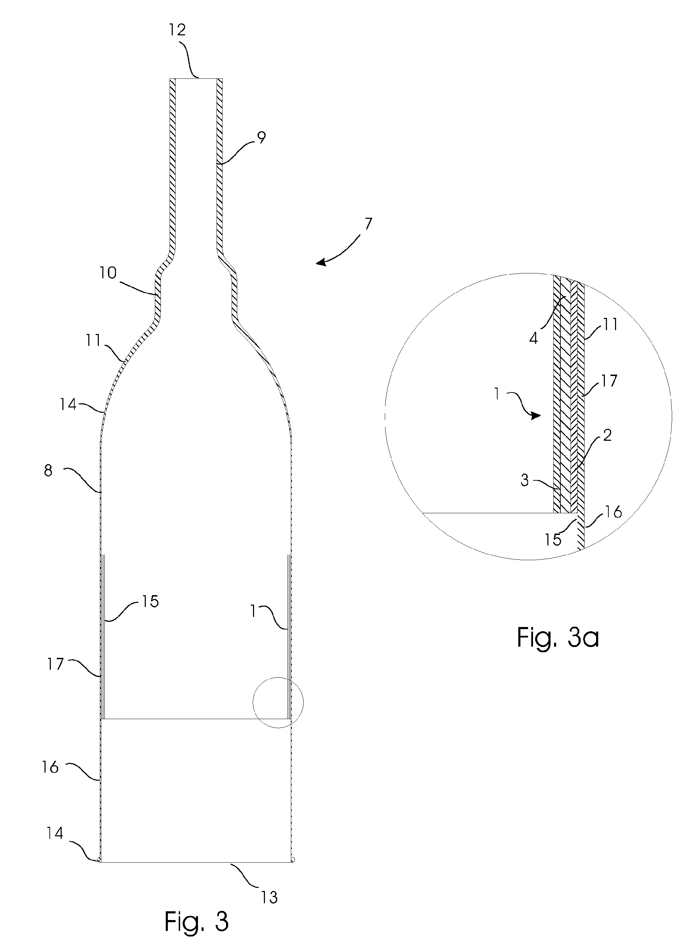

The adhesive laminate 1 appears as a part of the urisheath 7 on the inside 15 of the urisheath 7 as seen more clearly in the longitudinal sectional view of fig. 3 The adhesive laminate 1 constitutes a continuous annular adhesive liner or band which is strongly bonded to an annular middle section 17 of the tubular body part 8. The thickness of the wall 11 of the urisheath 7 is smallest at the tubular body part 8, which is in direct contact with the penile skin to provide an urisheath 7, which is particular comfortable to wear and is very little noticeable to the patient. The wall thickness of the remaining parts of the urisheath 7 is larger due to the fact that these parts are exposed to various external forces, including a continuous urine exposure and tensile loads when the patient moves. Within the scope of the present invention the adhesive laminate 1 can also be bonded to an urisheath having uniform wall thickness along its entire longitudinal length.

The structural arrangement of the layers 2,3,4 of the adhesive laminate 1 and the bonding to the inside 15 of the annular section 17 of the urisheath 7 is clear from the enlarged view of the circular outlined detail of fig. 3a. and will be further illustrated by way of the following Examples.

### Examples of Adhesive Laminates

### Example 1:

A two layer tape consisting of a 0.46 mm skin-friendly hydrocolloid based adhesive layer covered with a 0.03 mm smooth polyurethane backing film and provided on a paper liner is obtained from 3M Corporate Headquarters, 3M Center, St. Paul, MN 55144-1000 as product number 9943. The polyurethane backing film, which constitutes the carrier/barrier interlining for the laminate structure, is corona treated to obtain a surface tension of 72 mN/m and then bonded to silicone transfer adhesive, product number D752 obtainable from Specialty Tapes, Div. of RSW Inc., 4221 Courtney Lane, Franksville, WI 53126. The silicone transfer adhesive bonds directly to silicone without the use of corona treatment or any other oxidative process and a laminate structure composed of a first layer which adheres to cured silicone and a second layer which adheres to the skin. The polyurethane layer provides the laminate structure with structural integrity and serves as a carrier for the first and the second adhesive layer and at the same time serves as a barrier that prevents migration of molecules between the first and the second layer.

### Example 2:

A two layer tape consisting of a 0.46 mm skin-friendly hydrocolloid based adhesive layer covered with a 0.03 mm smooth polyurethane backing film and provided on a paper liner is obtained from 3M Corporate Headquarters, 3M Center, St. Paul, MN 55144-1000 as product number 9943. The tape is heated for 5 min at 100°C and then bonded at the free polyurethane surface to silicone transfer adhesive, product number D752 obtainable from Specialty Tapes, Div. of RSW Inc., 4221 Courtney Lane, Franksville, WI 53126. The silicone transfer adhesive bonds directly to silicone without the use of corona treatment or any other oxidative process and a laminate structure composed of a first layer which adheres to cured silicone and a second layer which adheres to the skin. The polyurethane layer provides the laminate structure with structural integrity and serves as a carrier for the first and the second adhesive layer and at the same time serves as a barrier that prevents migration of molecules between the first and the second layer.

### Example 3:

A hydrocolloid adhesive laminate manufactured by Euromed, 25 Corporate Drive, Orangeburg, NY 10962 is applied without any pre-treatment on top of a Platilon®U073 polyurethane film from Epurex Films GmbH & Co. KG, Bayershofer Weg 21, D29699 Bomlitz, Germany. On the other side is applied the same D572 silicone transfer adhesive from Speciality Tapes as in Example 1, to obtain a laminate structure in the form of a double sided tape having a side with a first layer which adheres to cured silicone and a side with a second layer which adheres to the skin.

### Example 4:

In a first coating step a Scotchpak 1022 release liner from 3M Corporate Headquarters, 3M Center, St. Paul, MN 55144-1000 is coated on one side with Bio-PSA 7-4301 silicone adhesive obtainable from Dow Corning Corporation, Corporate Center, PO box 994, Midland MI 43686-0994, USA at a coating weight of 45 g/m². Polyurethane film product number 3410 obtainable from Bemic Company Inc., One Neenah Center, Neenah, WI 54957, USA is applied on top of the silicone adhesive, and the polyurethane carrier/barrier film is corona treated as in Example 1.

In a second coating step a 42# Kraft release liner obtainable from Loparex OY, FI-08100 Lohja is coated with Durotak 80-192A acrylic adhesive obtainable from National Starch, 742 Grayson Street, Berkeley, CA 94710-2677. The acrylic adhesive is mated to bond to the corona treated polyurethane film to obtain a laminate structure in the form of a double sided tape having a side with a first layer, the silicone adhesive, which adheres to cured silicone and a side with a second layer, the acrylic adhesive, which adheres to the skin and not to cured silicone.

The final product is a tape having opposing adhesive sides covered by release liners.

The adhesive laminate provides various solutions for avoiding adhesion of the inside face to the outside face of male urisheaths which are rolled into windings before use. The adhesive laminate can be used as a separate part for adhering a conventional silicone rubber urisheath to a penis. The adhesive liner can also be provided as a firmly secured part on the inside of the urisheath, in which case an urisheath is provided which can be rolled so that the adhesive layer on the inside face are brought into contact with the outside face of the urisheath without the windings of the rolled urisheath are bonded unreleasbly to each other and no adhesive residues are left on the outside face.

The adhesive laminate according to the present invention can be employed in any design of catheters and e.g. applied to the inside of a catheter in a manual manufacturing process if preferred in preference to automated manufacturing methods.

## Claims

1. An adhesive laminate (1) adapted for bonding to an inside (15) of an annular section (17) of a male external catheter (7) of a cured elastomeric polymer, the adhesive laminate (1) is of the kind which comprises a first adhesive layer (2) directly bonded to the inside (15) of the elastomeric polymer wall (11) and a second adhesive layer (3) for securing the catheter (7) to the skin,
**characterized in that** the adhesive laminate (1) further comprises at least one third carrier and/or barrier layer (4) inserted between the first adhesive layer (2) and the second adhesive layer (3).

2. An adhesive laminate (1) according to claim 1, **characterized in that** the elastomeric polymer is a silicone rubber.

3. An adhesive laminate (1) according to any of the claims 1 or 2, **characterized in that** the second adhesive (3) is selected from a group of adhesive materials which selectively adheres to a skin surface but not stick to the elastomeric polymer or sticks to the elastomeric polymer in a releasable manner.

4. An adhesive laminate (1) according to any of the preceding claims 1, 2 or 3, **characterized in that** the least one third carrier and/or barrier layer (4) is made of a material that prevents molecular migration between the first (2) and second adhesive layer (3).

5. An adhesive laminate (1) according to any of the preceding claims 1 - 4, **characterized in that** the material of the at least one third carrier and/or barrier (4) layer is a an elastic polymer, preferably a thermoplastic polymer such as a polyurethane.

6. An adhesive laminate according (1) to any of the preceding claims 1 - 5, **characterized in that** the second adhesive layer (3) comprises at least one of the adhesive substances selected from the group comprising acrylic adhesive, polyurethan adhesives or hydrocolloids.

7. An adhesive laminate (1) according to any of the preceding claims 1 - 6, **characterized in that** at least one of the first adhesive layer (2) and/or the second adhesive layer (3) are covered by a release liner (5,6).

8. A method of adhering a male external urinary catheter (7) to a penis of the kind comprising a first tubular body part (8) which extends into a second tubular discharge part (9), **characterized in that** the adhesive laminate (1) according to any of the preceding claims 1 - 7 is adhered to the penis by means of the second adhesive (3) and subsequently an inner annular wall section (17) of the male external urinary catheter (7) is adhered to the first adhesive layer (2) of the adhesive laminate (1).

9. A method according to claim 8 **characterized in that** the male external urinary catheter (7) is detachable arranged on the adhesive laminate (1).

10. A male external urinary catheter (7) of the kind comprising
- a first tubular body part (8) provided with an adhesive means (1) for adhering to a penis,
- said first tubular body part (8) extends into a second tubular discharge part (9), **characterized in that** the adhesive means for adhering the first tubular part (8) to a penis is the adhesive laminate (1) according to any of the preceding claims 1 - 7.
